# EUROPEAN PATENT SPECIFICATION

(11) **EP 2 021 172 B1**
(45) Date of publication and mention of the grant of the patent: **26.05.2010**
(21) Application number: 07776859.6
(22) Date of filing: 07.05.2007
(51) Int. Cl.: B32B 3/18, C11D 3/50, C11D 17/04

(54) **FILMS WITH MICROCAPSULES**
FOLIEN MIT MIKROKAPSELN
FILMS AVEC MICROCAPSULES

(30) Priority: 05.05.2006 US 798158 P
(43) Date of publication of application: 11.02.2009
(73) Proprietor: The Procter and Gamble Company, Cincinnati, Ohio 45202 (US)
(72) Inventor: BROWN, Jodi, Lee, Cincinnati, Ohio 45208 (US); CATALFAMO, Vincenzo, Cincinnati, Ohio 45236 (US); WAHL, Errol, Hoffman, Cincinnati, Ohio 45242 (US); ARCHBOLD, James, Michael, West Chester, Ohio 45069 (US)
(74) Representative: Mather, Peter Geoffrey
(86) International application number: PCT/US2007/011063
(87) International publication number: WO 2007/130684

(56) References cited:
- WO-A-02/083043
- CA-A1- 2 524 099
- US-A- 4 528 226
- US-A1- 2005 013 990

## Description

### Technical Field

The present invention relates to functionalized substrates and films.

### Background of the invention

Consumers have come to expect a pleasant scent experience when laundering their clothing. There is a continuing need to provide consumers ways of enhancing their scent experience. See e.g., WO 2005/005591 A1; WO 2004/020566 A1. In spite of many advancements, there still exists a consumer need for more intense freshness on fabrics, different characters of freshness on fabrics, and especially, much longer lasting freshness on fabrics.

### Summary of the Invention

The present invention attempts to address this and other needs by providing an article comprising a functionalized substrate comprising at least two substantially planar water-soluble films carrying a coating of a functional composition, wherein the coating is between two films, and wherein the functional composition comprises at least a microcapsule as defined in claim 1. The microcapsule comprises a perfume microcapsule. Articles comprising perfume can be used as a scent booster to provide increased dry fabric odor when used in addition to a laundry detergent. Likewise, the article can be used as a scent booster to provide increased dry fabric odor when used in addition to a fabric conditioning product. In addition to increased dry fabric odor initially, the use of the article can provide a much longer lasting fabric scent. The article may be added to the wash cycle, the rinse cycle or both. Other functional benefits are possible; for example the article could provide additional softness, static control or bleach activity. In another embodiment, the article can provide a means of customization by the consumer. The article can be used to provide most or all of the scent to the fabrics when the consumer uses the article with an unscented detergent, and/or an unscented fabric conditioner.

A second aspect of the invention provides for a process for making a functionalized substrate in the form of a water-soluble film carrying a coating of a functional composition, wherein the functional composition comprises a microcapsule, the process comprising printing to at least one side of the film to form the coating. Other functional compositions may also be imparted to the film in addition to a microcapsule

### Detailed Description

According to a first aspect of the invention, there is provided a process for making a substrate in the form of a water-soluble film carrying a coating of a functional composition. By "functional composition" is herein meant a composition which comprises one or more materials that performs a function or delivers a benefit after dissolution of the film or which modify the physical or chemical properties of the film, other than aesthetic appearance. For example inks, decorative dyes and pigments are not considered functional materials. However, a hueing dye for improved whiteness appearance of fabrics is considered functional.

The process of the invention is suitable for loading high levels of one or more functional materials. The coating is in a level of at least 5, alternatively at least 10, alternatively at least 50, and alternatively at least 100 g/m². In another embodiment, the loading is at least about 10%; alternatively at least about 20%, alternatively at least about 30%, alternatively at least about 40%, alternatively at least about 50%, alternatively at least about 100%, and alternatively at least about 200%, alternatively not greater than 1000%, by weight of the uncoated film. In yet another embodiment, the coating comprises the one or more functional materials in a level of at least about 10%, alternatively at least about 20%, alternatively at least about 30%, alternatively at least about 40%, alternatively at least about 50%, alternatively at least about 60%, alternatively at least about 70%, alternatively at least about 80%, by weight of the dried coating.

Substrates obtained according to the process of the invention are a very efficient way of delivering high levels of functional materials in water to the wash and/or rinse cycles because the thickness of the film does not limit the amount of materials that can be loaded (i.e., high loadings of active material or materials can be achieved). The films can be loaded with a wide variety of functional materials, including, but not limited to: flavors, perfumes, softening agents, anti-static agents, crisping agents, water/stain repellents, stain release agents, refreshing agents, anti-microbial agents, disinfecting agents, wrinkle resistant agents, wrinkle release agents, odor resistance agents, malodor control agents, abrasion resistance and protection agents, solvents, insect/pet repellents, wetting agents, UV protection agents, skin/fabric conditioning agents, skin/fabric nurturing agents, color protection agents, dye fixatives, dye transfer inhibiting agents, silicones, preservatives and anti-microbials, fabric shrinkage-reducing agents, perfume microcapsules, brighteners, hueing dyes, bleaches, chelants, antifoams, anti-scum agents, whitening agents, and combinations thereof. Microcapsules containing these and other commonly used fabric conditioning actives can be used in accordance with the present invention.

The process comprises the step of applying to at least one side of the water-soluble film a solution, optionally an aqueous solution, comprising one or more functional material(s) to form the coating wherein the coating is formed from one or more layers in a stepwise manner. In one embodiment the solution comprises a film insolubilizer agent.

By "aqueous solution" is herein meant a solution in which the solvent in major proportion is water. The aqueous solution can also comprise other solvents in minor proportions. In one embodiment, the water content of the aqueous solution is at least about 10%, alternatively at least alternatively 20%, alternatively at least about 30%, alternatively at least about 40%, alternatively not greater than 99% by weight above the level of any other solvent present in the aqueous solution. In another embodiment, the water content of the aqueous solution is at least about 20%, alternatively at least about 30%, alternatively at least about 40%, alternatively at least about 60%, alternatively not greater than 99% by weight of the aqueous solution. The term "aqueous solution" should be broadly interpreted for the purpose of this invention, including any mixture comprising water and functional material. Slurries and dispersions (liquid/solid), foams (liquid-gas), gels, and emulsions (liquid/liquid) are considered to be "aqueous solutions."

The term "solution" as used herein, means aqueous solutions or non-aqueous solutions. Non-aqueous solutions means a solution is which the water content, if any, is below about 10%, alternatively below about 5%, alternatively below about 3%, alternatively below about 1%, alternatively substantially free of water, alternatively free of water, by weight of the non-aqueous solution. Non-aqueous solutions may include solvents such as glycols (e.g., propylene glycol, polyethylene glycol, and gylcerin) or waxes. In another embodiment no solution is used, but a powder or solid active alone or in a powder/solid carrier is coated onto the film.

One of the advantages of the process of one embodiment of the present invention is that it may not require the use of organic solvents which are expensive, difficult to handle and have environmental and safety risks associated with them. One of the challenges faced by a process using an aqueous solution to treat a water-soluble, i.e., water-sensitive film, is that the film is susceptible to water attack. The film could be degraded (i.e., formation of pin holes, shrinkage, deformation, formation of visible ribs, sagging, thinning-out, etc.) and some of its initial properties could be lost if a drying step is performed as soon as possible after the film has been exposed to the aqueous solution. In one embodiment of the invention, an aqueous solution is used to treat the water-soluble film, but does not substantially alter the properties of the water-soluble film and does not substantially change the water content of the water-soluble film with respect to the uncoated film.

Without being bound by theory, it is believed that the entire surface, or a discrete part thereof, of the water-soluble film may partially be dissolved or solvated by the aqueous solution. Once the water from the aqueous solution has been removed from the surface of the film, it resolidifies thereby adhering the functional material onto it. Thus, another potential advantage of the process of one of the embodiments of the invention is that the coating may be adhered or fixed to the film without the use of binders or other auxiliary agents. This reduces the cost and simplifies the process.

### Coating

In one embodiment of the present invention, the surface of the film is at least partially coated with a functional material. In one embodiment, the film comprises two planar faces wherein at least one of the faces is at least partially coated with a functional material. In another embodiment, a discrete portion of the film is coated with the functional material. Treatment in discrete portions of the film may be desired in order to avoid, for example, exposure to harmful transformations during converting the film into a packaged product, such as a pouch or sachet. For example, when heat sealing a film which has been coated with a flammable functional material, such as flammable perfume, it may be desirable to coat a discrete portion of the film so uncoated areas of the film can be heat treated. In another embodiment, the article comprises a fully coated functional substrate, wherein at least one of the faces of the substrate is fully coated with a functional material.

In one embodiment, the film coating comprises a uniform pattern of coating, wherein the coating is uniform across the area coated. In another embodiment, the film coating comprises a random pattern of coating, wherein the coating is not uniform across the area coated.

When the coating is formed from a plurality (e.g., two, three, four, five, six, or more) of layers in a stepwise manner, the first layer, (i.e., the layer in direct contact with the film), is relatively thin or is dried at substantially the same time as the aqueous solution is placed on the film. In order to determine the thickness of the first layer and/or the rate of drying of this layer, trial and error could be used. The thickness, rate of drying and any other variables of the process should be such as to maintain the mechanical integrity of the film. This can be tested by measuring the elasticity of the film before and after the first layer has been formed. The elastic properties of the film (tensile strength, elongation modulus and percentage of elongation at break) should be within about 40%, alternatively within about 30%, alternatively within about 20%, alternatively within about 10% of those of the uncoated film when measured under identical relative humidity and temperature conditions; for example at 40% relative humidity and 20°C, preferably the film is kept at these conditions for 24 hours before the measure is performed.

The use of a stepwise process allows partial or total drying to take place before the next layer is deposited thereby avoiding the exposure of the film to extremely high levels of water all at once. In one embodiment, there are no limitations on the amount of water that the aqueous solution can comprise. The first layer protects the water-soluble film from interactions with the successive layers. An additional benefit of applying multiple thin layers of the aqueous solution on the water-soluble film as opposed to large quantities altogether, is that the drying steps can be accomplished under mild drying conditions, i.e., short ovens, small air flows, lower temperatures, simpler method (e.g., hot air vs. IR) and hence more economically. Furthermore, mild drying conditions may make the process suitable for heat sensitive ingredients such as enzymes, perfumes, bio-actives (e.g. proteins, catalysts and vitamins) etc.

The coating comprising the functional material(s) can be formed from a solution comprising a film insolubilizer, i.e., an agent that temporarily reduces the solubility of the film in presence of the aqueous solution at the level at which is used in the process. However, the functionalized film remains soluble when immersed in water. The water-soluble film is less prone to water attack (still gets wet by the aqueous solution so the functional material(s) can be deposited but it does not get solubilized or does not absorb water in depth causing film swelling and alteration of physical properties). This may allow the using of layers of greater thickness and consequently can decrease the number of layers needed. For certain applications, it might be reduced to just one. The film insolubilizer, in one embodiment, may be applied before the aqueous solution comprising the functional material or as part of the aqueous solution. In another embodiment, the film insolublizer may delay full dissolution of the substrate until later in the wash and/or rinse cycle, thereby potentially improving the deposition of functional materials onto the fabrics.

The coating can be applied on the film by means of any coating process, including spray, knife, rod, kiss, slot, painting, printing and mixtures thereof. Printing is typically done with inks and dyes and used to impart patterns and colors to substrates. In the case of the present invention, printing is used to deposit the functional material(s) onto a water-soluble film. Any kind of printing can be used, for purposes of the present invention, including rotogravure, lithography, flexography, porous and screen printing, inkjet printing, letterpress, tampography and combinations thereof. In one embodiment, the coating is applied by flexography printing. Flexography printing equipment is relatively inexpensive and runs fast in comparison with other printing techniques. An advantage of flexography printing is the common multi-printing stations set-up so that multiple printing can be accomplished in one pass with ordinary equipment. Another advantage of flexographic printing is its flexibility to handle printing solutions of high viscosity and wider particle size range than generally ink jet printing. Flexography is a printing technology which uses flexible raised rubber or photopolymer plates to carry the printing solution to a given substrate. In one aspect of the invention, the flexible plates carry the aqueous solution to the film. The fact that the aqueous solution is water based does not give rise to incompatibilities with the plate which can cause the plate to swell thereby impairing in the accuracy or resolution of the printing.

In one embodiment, the process comprises the step of depositing a second film over the coating and sealing the two films to form a laminate. These embodiments are especially suitable when the coating comprises functional materials that should be protected from the surrounding environment due to incompatibility issues or stability issues or that should be isolated in order to avoid the contact with the skin of the user. The second film can also comprise a coating of a functional composition. In yet another embodiment, a third film (with or without a coating of a functional composition) may also be included to comprise the laminate. The functional composition of the second film or the third film may be different from the functional composition of the first film. The number of films sealed together is determined by the application of the functionalized film. Alternate sealing methods include heat, solvents, ultrasonics, infrared, or combinations thereof.

### Microcapsule

In one embodiment, the functional material(s) comprises a microcapsule encapsulating an active. In one embodiment, the active comprises a fabric care active. In another embodiment, the active comprises silicone oils and silicone waxes, waxes, hydrocarbons, higher fatty acids, essential oils, lipids, skin coolants, vitamins, sunscreens, antioxidants, glycerin, catalysts, bleach particles, silicon dioxide particles, malodor reducing agents, dyes, brighteners, antibacterial actives, antiperspirant actives, cationic polymers or a mixture thereof. In one aspect, the perfume raw material comprises alcohols, ketones, aldehydes, esters, ethers, nitriles alkenes, or a mixture thereof. In one aspect the core material comprises a perfume. In another embodiment, the microcapsule core contains PDMS or derivatized PDMS (one example is silicone polyols), aminofunctional silicones, sucrose polyesters, polyglycerol esters, polyethylene waxes, Vitamin E, enzymes, amino acids, Shea Butter, aloe vera, petrolatum, retinol, Cucumber Extracts, Chamomile Extracts, Almond Milk, Silk Protein, keratin protein and keratin amino acids, Natural Soap, eucalyptus, Natural Oat, sea minerals, lavender, rose, Vanilla Extract, Linen Flower, citrus, lemon, lime, and orange.

Other functional materials include laundry cleaning actives, barrier agents, solubility modifiers, fabric softening actives, silicones, antifoams and mixtures thereof.

The term "microcapsule" is used herein the broadest sense and includes the encapsulation of perfume or other materials or actives in small capsules (i.e., microcapsules), typically having a diameter less than 300 microns. Typically, these microcapsules comprise a spherical hollow shell of water insoluble or at least partially water insoluble material, typically polymer material, within which the active material, such as perfume, is contained. Microcapsules are described in the following references: US 2003/215417 A1; US 2003/216488 A1; US 2003/158344 A1; US 2003/165692 A1; US 2004/071742 A1; US 2004/071746 A1; US 2004/072719 A1; US 2004/072720 A1; EP 1,393,706 A1; US 2003/203829 A1; US 2003/195133 A1; US 2004/087477 A1; US 2004/0106536 A1; US 6,645,479; US 6,200,949; US 4,882,220; US 4,917,920; US 4,514,461; US RE 32,713; US 4,234,627.

Microcapsules may be prepared using a range of conventional methods known to those skilled in the art for making shell capsules, such as interfacial polymerization, and polycondensation. See e.g., US 3,516,941, US 4,520,142, US 4,528,226, US 4,681,806, US 4,145,184; GB 2,073,132; WO 99/17871; and MICROENCAPSULATION: Methods and Industrial Applications Edited by Benita and Simon (Marcel Dekker, Inc. 1996). It is recognized, however, that many variations with regard to materials and process steps are possible. Non-limiting examples of materials suitable for making shell of the microcapsule include urea-formaldehyde, melamine-formaldehyde, phenol-formaldehyde, gelatin, polyurethane, polyamides.

In one embodiment of the invention, the shell of the microcapsules comprises an aminoplast resin. A method for forming such shell capsules includes polycondensation. Aminoplast resins are the reaction products of one or more amines with one or more aldehydes, typically formaldehyde. Non-limiting examples of suitable amines include urea, thiourea, melamine and its derivates, benzoguanamine and acetoguanamine and combinations of amines. Suitable cross-linking agents (e.g., toluene diisocyanate, divinyl benzene, butanediol diacrylate etc.) may also be used and secondary wall polymers may also be used as appropriate, e.g., anhydrides and their derivatives, particularly polymers and co-polymers of maleic anhydride as disclosed in US 2004/0087477 A1. In another embodiment, the shell of the microcapsules comprise urea-formaldehyde; melamine-formaldehyde; or combinations thereof.

The microcapsules of the present invention, in one embodiment, are friable in nature. Friability refers to the propensity of the microcapsules to rupture or break open when subjected to direct external pressures or shear forces. For purposes of the present invention, the microcapsules utilized are "friable" if, while attached to fabrics treated therewith, they can be ruptured by the forces encountered when the capsule-containing fabrics are manipulated by being worn or handled (thereby releasing the contents of the capsule). In an another embodiment, the microcapsules are combinations of friable microcapsules and moisture-activated microcapsules. In yet another embodiment, the microcapsules used in the present invention are or combinations of friable, moisture-activated, and heat-activated microcapsules.

In one embodiment, the shell capsules typically have a mean diameter in the range 1 micrometer to 100 micrometers, alternatively from 5 micrometers to 80 micrometers, alternatively from 10 micrometers to 75 micrometers, and alternatively between 15 micrometers to 50 micrometers. The particle size distribution can be narrow, broad or multimodal.

In another embodiment, microcapsules vary in size having a maximum diameter between about 5 microns and about 300 microns, alternatively between about 10 microns and about 200 microns. As the capsule particle size approaches 300 microns, e.g. 250 microns), a reduction in the number of capsules entrained in the fabric may be observed.

In another embodiment, the capsules utilized in the present invention generally have an average shell thickness ranging from about 0.1 micron to 50 microns, alternatively from about 1 micron to about 10 microns.

In another embodiment, the microcapsules comprise a loading/complexation level of from about 50% to about 90%, alternatively from about 60% to about 85%, alternatively from about 65%% to about 75%, by weight of a perfume composition. This loading/complexation property of the perfume microcapsules of the present invention is advantageous versus other technologies such as beta-cyclodextrin. The advantages may include, but are not limited to, one or more of the following: (i) the ability to use a reduced total perfume level, e.g., in neat perfume (direct add); in perfume microcapsules; or combinations thereof; (ii) avoiding cost in processing and lost material through processing; (iii) delivering a high level of perfume while not affecting process product disposition or process parameters; and (iv) delivering a high level of perfume to fabric while avoiding a high level of neat product odor, which can be a consumer negative; and (v) delivering improved fabric odor longevity performance compared to neat perfume.

Suppliers of microcapsules may include International Flavors & Fragrances (IFF), Reed Pacific, and Appleton. An example of a suitable microcapsule for purposes of the present invention includes Perfume Microcapsules (PMCs) from Appleton. Other examples may include WIZARD from Reed Pacific, and EVERLAST from IFF. For one embodiment, the shell is formed by cross-linking aldehydes and amine functionalities. In one embodiment, the encapsulated blooming perfume composition may, in one embodiment, comprise from about 3 to about 300 different perfume ingredients, optionally with minimal modifiers which include viscosity or hydrophobicity modifiers. Typical viscosity modifiers include, but not limited to, silicone oil, gums, and waxes. Typical hydrophobic modifiers include, but not limited to, isopropyl myristate, mineral oil, dipropylenemethyl ether (DPM). Such modifiers may be used at less than 50%, alternatively less than 40%, alternatively less than 30%, alternatively less than 20%, alternatively less than 10%, alternatively less than 5%, alternatively less than 1%, alternatively about 0%, alternatively at least 0.1 % but not greater than 50%, by weight of total perfume composition. Without wishing to be bound by theory, the overuse of modifiers reduces the efficiency of the scent experience imparted by the perfume microcapsules of the present invention.

Once microcapsules containing a perfume composition of the present invention have been attached to fabrics being treated, it is, of course, necessary to manipulate the treated fabrics in a manner sufficient to rupture the microcapsules and thereby release the perfume composition. Microcapsules of the type utilized herein have friability characteristics such that the ordinary fabric manipulation which occurs when the treated fabrics are worn or used is sufficient for the attached microcapsules to impart a noticeable odor to the fabric. A significant number of attached microcapsules can be broken by the normal forces encountered when treated garments are worn. For fabric articles which are not worn, the normal household handling operations such as folding, crumpling etc. can serve as fabric manipulation sufficient to rupture the attached microcapsules. The perfume composition used in the present invention surprisingly maximizes the effect of the microcapsules bursting by providing a perfume composition that "blooms" upon the microcapsules rupturing.

The friable microcapsules used in the present invention are distinguished from moisture-activated microcapsules, such as those capsules comprising of cyclodextrin that burst upon contact with moisture; a wax comprising microcapsule such as those described in U.S. Pat. No. 5,246,603; and starch-based microcapsule also described in U.S. Pat. No. 5,246,603.

### Blooming Perfume

The present invention is based, in part, upon the discovery that the blooming perfume compositions of the present invention maximizes the opportunity for the consumer of a unique scent experience during the wearing, folding, and even after storage of laundry when fabric deposited with friable microcapsules are ruptured. In one embodiment, the perfume microcapsule encapsulates a blooming perfume composition, wherein the blooming perfume composition, in the absence of water, comprises from about 5% to about 95%, alternatively from about 20% to about 90%; alternatively from about 30% to about 85%, and alternatively from about 40% to about 80%, by the total weight of the perfume microcapsule and the encapsulated perfume composition, also in absence of water.

The term "blooming perfume composition" as used herein means a perfume composition that comprises at least about 25%, alternatively at least about 35%, alternatively at least about 45%, alternatively at least about 55%, alternatively at least about 65%, by weight of the perfume composition, of blooming perfume ingredients, wherein the blooming perfume ingredients are those having a boiling point (B.P.) equal to or lower than about 250°C, alternatively equal to or lower than about 250°C, wherein the B.P. is measured at the normal standard pressure.

The boiling points of many perfume ingredients are given in, e.g., "Perfume and Flavor Chemicals (Aroma Chemicals)," S. Arctander, published by the author, 1969. Other boiling point values can be obtained from different chemistry handbooks and databases, such as the Beilstein Handbook, Lange's Handbook of Chemistry, and the CRC Handbook of Chemistry and Physics. When a boiling point is given only at a different pressure, usually at a pressure lower than the standard pressure (760 mm Hg), the boiling point at standard pressure can be approximately estimated by using boiling point-pressure monographs, such as those given in "The Chemist's Companion," A. J. Gordon and R. A. Ford, John Wiley & Sons Publishers, 1972, pp. 30-36. When applicable, the boiling point values can also be calculated by computer programs, based on molecular structural data, such as those described in "Computer-Assisted Prediction of Normal Boiling Points of Pyrans and Pyrroles," D. T. Stanton et al, J. Chem. Inf. Comput. Sci., 32 (1992), pp. 306-316, "Computer-Assisted Prediction of Normal Boiling Points of Furans," Tetrahydrofurans, and Thiophenes," D. T. Stanton et al, J. Chem. Inf. Comput. Sci., 31 (1992), pp. 301-310, and references cited therein, and "Predicting Physical Properties from Molecular Structure," R. Murugan et al, Chemtech, June 1994, pp. 17-23.

Non-limiting examples of blooming perfume ingredients that are useful in the articles of the present invention are given in U.S. Pat. Pub. No. 2005/0192207 A1, published Sep. 1, 2005, ¶¶29 - 31.

In one embodiment, the blooming perfume compositions used in the present invention comprises at least about 3 different blooming perfume ingredients, alternatively at least about 4 different blooming perfume ingredients, alternatively at least about 5 different blooming perfume ingredients, and alternatively at least about 6 different blooming perfume ingredients.

In the perfume art, some materials having no odor or very faint odor are used as diluents or extenders. Non-limiting examples of these materials are dipropylene glycol, diethyl phthalate, triethyl citrate, isopropyl myristate, and benzyl benzoate. These materials are used for, e.g., diluting and stabilizing some other perfume ingredients. For purposes of this invention, these materials are not counted as a "blooming perfume ingredient."

In one embodiment, substantive perfume ingredients, which can be used as part of blooming perfume compositions in articles of the present invention, are those having a B.P. higher than about 250°C. Non-limiting examples of such perfume ingredients include those described in U.S. Pat. Pub. No. 2005/0192207 A1, published Sep. 1, 2005, ¶36.

Another aspect of the invention provides for function composition to comprise an optional perfume component comprising at least one of the following: (a) a moisture-activated perfume microcapsule comprising a perfume carrier and an encapsulated perfume composition; (b) a pro-perfume; (c) a low odor detection threshold perfume ingredients; (d) neat perfume; and (e) combinations thereof. In one embodiment, the article is free or substantially free of any one or more of the aforementioned perfume components. A non-limiting example of a moisture-activated perfume microcapsule includes one that comprises cyclodextrin.

### Laundry Cleaning Actives

Laundry cleaning actives are substances which play an active role in the cleaning process of laundry. Cleaning actives include, but are not limited to, substances such as detersive surfactants (anionic, nonionic, cationic and amphoteric surfactants), builders (inorganic and organic builder substances), bleaches, bleach activators, bleach stabilizers, bleach catalysts, enzymes, or combinations thereof, without the term being restricted to these substance groups. In one embodiment, the term "cleaning active" may be free or substantially free of one or more above identified actives. In one embodiment, the laundry cleaning active is encapsulated in the microcapsule.
Barrier agents perform a protective function. For example, they can protect mutually incompatible cleaning actives from one another, cleaning actives or solubility modifiers from the outside environment, the film from the external environment, etc. They can also modify the feeling at touch of the film and/or functional materials. They can make substrates more pleasant to the touch. Suitable barrier agents may include zeolite, bentonite, talc, mica, kaolin, silica, silicone, starch cyclodextrin, varnish, shellac, lacquer, polyolefins, paraffins, waxes, polyacrylates, polyurethanes, polyvinyl alcohol, polyvinyl acetate, UV absorbers ( see e.g., McCutcheon Volume 2, Functional Materials, North American Edition, published by the Manufacturing Confectioner Publishing Company (1997)), fluorescent dyes, (see e.g., EP 1,141,207, US 5,082,578), or combinations thereof.

### Solubility Modifiers

Solubility modifiers are substances which modify the solubility of the film and/or functional materials by for example delaying or accelerating its solubility or making solubility dependent of external factors such as pH, temperature, ionic strength, redox potential, etc. One example of a solubility modifier is an amino-acetylated polysaccharide, optionally chitosan, having a selected degree of acetylation. The solubility of chitosan is pH dependent and in one embodiment, the dissolution of the functionalized substrate is restricted to a determined pH by making use of this property. Other suitable solubility modifiers may include the polymers described in US 2003/0158072 A1, whose water solubility may be triggered by changes in pH, salt concentration, concentration of surfactant or a combination of both. The polymer is a copolymer or terpolymer containing from 2 to 60 mole percent of a protonated amine functionality which has been neutralized with a fixed acid. WO 02/26928 provides non-limiting examples of suitable composite polymers that can be used for controlled release purposes, as in laundry.

Additional suitable solubility modifiers that are soluble in a given pH range are based on methacrylic acid co-polymers, styrene hydroxystyrene co-polymers, acrylate co-polymers, polyethylene glycol polyvinyl acetate, diethylphtalate, dioctyl sodium sulfocuccinate, poly-dl-lactide-co-glycolide (PLG), vinylpyridine/styrene co-polymers, chitosan/lactic acid, chitosan/polyvyl acohol, commercially available from Degussa Rhom Pharma under the trade name Eudragit, from Eastman under the trade name Eastacryl, from MacroMed Inc. under the trade name SQZgel.

Solubility modifiers that are soluble in a specific chemistry environment are also commercially available. For instance caustic soluble barrier agents are commercially available from Alcoa under the trade name Hydra-Coat-5. Water dispersible barrier agents are based on Sodium starch glycolate, polyplasdone and are commercially available from FMC Corporation under the trade name Ac-di-sol, from Edward Mendell Corporation under the trade name Explotab, from ISP under the trade name Crospovidone.

In one embodiment, the functional composition may comprise one or more of the following material(s): soil release polymer, anti-oxidants, colorants, preservatives, optical brighteners, opacifiers, stabilizers such as guar gum and polyethylene glycol, anti-shrinkage agents, anti-wrinkle agents, soil release agents, fabric crisping agents, reductive agents, spotting agents, germicides, fungicides, anti-corrosion agents, antifoam agents, hueing dyes, and the like. In one embodiment, the functional composition is free or substantially free of any one or more of the above-identified optional components.

In another embodiment the functionalized substrate further comprises an aesthetic agent. The aesthetic agent can have ornamental purposes and can denote the presence of functional materials on the film. It can also signal when a functional material is released or a product "end of life" via a change in colour and/or appearance/disappearance of graphics, patterns, etc.

In another embodiment, the perfume comprises from about 1% to about 99% by weight of the coating. Alternatively, the perfume comprises at least about 5%, alternatively 15%, alternatively 25%, alternatively 40%, alternatively 70%, alternatively 85%, but not greater than 99.9%, by weight of the coating. In yet another embodiment, the perfume comprises from about 1% to about 99% by weight of the laminate. Alternatively, the perfume comprises at least about 5%, alternatively 15%, alternatively 25%, alternatively 40%, alternatively 70%, alternatively 85%, but not greater than 99.9%, by weight of the laminate. The term "perfume," for purposes of this paragraph means both neat perfume and perfume encapsulate within the microcapsule (but not the shell of the microcapsule) or any other perfume bound to a perfume carrier (but not he perfume carrier itself). For purposes of clarification, the coating and laminate are measured as they are or would be sold in commerce.

### Functionalized Substrates

According to a product aspect of the invention, there is provided a functionalized substrate in the form of a water-soluble film carrying a coating of a functional composition. The functional composition comprises one or more functional material(s). The coating is in a level of at least about 5, alternatively at least about 10, alternatively at least about 25, alternatively at least about 50, alternatively at least about 70, alternatively at least about 100 g/m²; but alternatively not greater than 1 kg/m². The coating is at a loading of at least about 30%, alternatively at least about 50%, alternatively at least about 100%, alternatively at least about 200% by weight of the uncoated film; but alternatively not greater than 1,000%. In one embodiment, the functionalized substrate is obtainable or obtained according to a process of the present invention.

The functionalized substrates of the present invention may have a multitude of applications. One application is in the field of fabric care. One method provides for administering the substrate into the basin of an automatic laundry washing machine, either in the wash cycle or the rinse cycle or both. Another method is directly administering the substrate before or during a wash cycle (opposed to a rinse cycle). Administering before the rinse cycle is typically more convenient to the user because she does not need to remember to come back during the rinse cycle. Yet another method includes administering two or more substrates to the washing machine. A user may administer more than one substrate based on her perfume or freshness desire. In yet another method, the user chooses a substrate that comprises a perfume that has the same scent (alternatively a complimentary scent) as the laundry detergent or fabric softening composition that she is also using. Instructions on the packaging for the functional substrates may instruct the user about one or more of these methods.

In one embodiment, the functionalized substrate is cut into or prepared in the form of small pieces, having, in one embodiment, a maximum linear dimension of from about 0.2 to about 100 mm, alternatively from about 0.5 to about 50 mm, and alternatively from about 1 to about 20 mm, as a stand alone product or as part of another product. The substrate may be cut or prepared into "confetti" that is added or incorporated as part of the powder, liquid or gel compositions, in another embodiment, the functionalized substrate is cut or formed into a shape which resembles, including but not limited to, flower petals or a leaves. Another embodiment can be in the form of a strip of film on a roll to dispense like tape with perforations to allow separation of segments of film. In one embodiment, the roll of film can be contained in a separate container or in a compartment of the closure; for example the closure of a bottle of liquid fabric conditioner and/or a liquid detergent product. Functionalized films are an effective way of protecting sensitive ingredients as well as controlling the delivery of functional materials. In order to provide additional protection, the cutting operation can be registered with the functional material application operation so that no functional material is potentially exposed on the edge of the cut pieces. This is particularly advantageous when the functionalized cut pieces are introduced in a product in liquid/gel form that can potentially react with the functional material exposed on the edge of the cut pieces.

In one embodiment of the present invention, the functionalized substrate comprises a shape, wherein the shape and the functional material are coordinated. By shape, it is also meant that the form of the functionalized substrate can include any drawings, wording and/or colorations which can be placed on the functionalized substrate by any method known in the art. This includes scent and shape names as well as trademarks. As used herein, coordinated means any relationship between the shape and the functional material such that a consumer would understand that the shape represents the functional material or that the functional material represents the shape. In one embodiment the functional material comprises a perfume. In one embodiment, the perfume provides a specific scent experience. In another embodiment, the shape of the functionalized substrate is coordinated with the scent experience generated by the perfume.

Non-limiting examples of coordinated shapes and scent experiences include any natural or artificial combinations of shape and scent. Naturally occurring combinations of shape and scent include, but are not limited to: rose, rose petal, lavender, lilac, sunflower, canola flower, daisy, tulip, daffodil, dahlia, honeysuckle, honey comb, morning glory, jasmine, water lily, lily, carnation, orchid, white orchid, lotus flower, magnolia, violet, pansy, orange, lemon, lime, apricot, tangerine, plum, mandarin, mango, kiwi, apple, peach, pear, cherry, grape, cucumber, lavender, vanilla, vanilla bean, coffee bean, aloe vera plant, chamomile, eucalyptus, peony, gardenia, white gardenia, ylang, lily of valley, hyacinth, linden blossom, osmanthus, tuberose, orange flower, persimmon, bergamot, banana, strawberry, blueberry, gooseberry, raspberry, blackberry, juniper berry, rhubarb, papaya, guava, passion fruit, grapefruit, white grapefruit, pink grapefruit, boysenberry, watermelon, honeydew melon, cantaloupe, pomegranate, tea leaves, white tea, pine/pinecone, cedar, maple leaf, oak, ash, elm, rain drop, slices of any of the above mentioned fruits or vegetables, ), palm tree, cherry blossom, clover, cinnamon stick, ivy, water drop, ocean wave, white lilac, lemon verbena, rice flower, ginger flower, cotton blossom, milk & honey, linen flower, and sweet pea. Artificial combinations of shape and scent include, but are not limited to: talcum powder scent with baby bundle, bottle, stuffed animals, linens, pillows, and bedding, bath and towel shapes; new car small with automobile shapes, morning dew scent with rain drops, sun, moon, star, or cloud shapes.

In one embodiment, small pieces of the substrate are incorporated as part of a laundry detergent product (e.g., dry, liquid or other form). Alternatively, small pieces of the substrate are incorporated as part of a liquid, rinse-added, fabric softening product. The small pieces may be substantially uniformly distributed throughout the composition of the product. The use of structurants or thickening agents in the composition is one way of keeping the small pieces of substrate suspended, substantially uniformly distributed throughout the composition. In one embodiment the liquid laundry detergent product and/or the liquid fabric softening product is clear or translucent. The clear or translucent liquid allows the small pieces of the substrate to be more highly visible. The laundry compositions may be contained in a multiple unit dose container (e.g., bottle) or single unit container that is clear, substantially clear, translucent, or substantially translucent, to showcase the small pieces in the composition to the user. In one embodiment, at least 10%, alternatively at least 20%, alternatively at least 30%, alternatively at least 40%, alternatively at least 50%, alternatively at least 75%, alternatively at least 90%, of the surface area of the container is clear, substantially clear, translucent, or substantially translucent; but alternatively not greater than 99.9%. Such materials include polyethylene terephthalate (i.e., PET, PETE, or PETP). The small pieces may be of uniform or substantially uniform shapes or size or may be different based upon product design. Small pieces may be in the shape of squares, triangles, rectangles, circles, or other geometric shapes, or completely random, or combinations thereof. The small pieces each may have same color or a different color.

The functionalized substrate is very well suited for use in unit dose fabric care products (such as pouches, capsules and sachets) either as part of the enveloping material or as part of the contents enclosed within the enveloping material. In one embodiment the enveloping material is formed at least in part of the functionalized substrate. For example, a single compartment unit dose form typically has separate bottom and top layers of enveloping material; according to this embodiment one or both layers can comprise or be composed of the functionalized substrate of the invention. The same is true for multi-compartment unit dose forms in which top, bottom and/or any of the intermediate layers of enveloping material can comprise or be composed of the functionalized substrate of the invention.

One aspect of the present invention provides a process for making a functionalized substrate by depositing a functional material(s). In one embodiment the depositing comprises printing the functional material onto a water-soluble film. The invention also envisages a functionalized substrate and fabric care products comprising the substrate of the invention. The process is capable of depositing high loads of functional material(s) using aqueous solutions without impairing or without substantially impairing the properties of the water-soluble film.

The functionalized substrate of the invention can be made by depositing a coating of a functional composition using suitable coating means including spraying, knife, rod, kiss, slot, painting, printing and combinations thereof. Printing is an optional method for use herein. In one embodiment, the printing is flexographic (flexo) printing. In the typical flexo printing sequence, the water-soluble film is fed into the press from a roll. The functional material is printed as the film is pulled through one or more stations, or print units. Each print unit can print the aqueous solution comprising one or more functional materials. Each printing step on a flexo press consists of a series of four rollers or cylinders: fountain roller, meter or anilox roller, flexographic or printing cylinder and impression cylinder.

The first roller (fountain roller) transfers the aqueous solution comprising the functional material(s) from the solution pan to the meter or anilox roller, which is the second roller. A doctor blade may be used if it is necessary to scrape some of the aqueous solution. The anilox roller meters the aqueous solution to a uniform thickness onto the printing cylinder. The substrate then moves between the printing cylinder and the impression cylinder, which is the fourth roller. In some flexographic equipment the fountain roller is missing and the anilox roller functions as both the fountain roller and the meter roller.

The impression cylinder applies pressure to the printing cylinder, thereby transferring the functional material(s) onto the film. The printed film may be fed into an overhead dryer so the newly formed layer is dried to remove most of the residual water before it goes to the next print unit. The finished product is then rewound onto a roll or is fed through the cutter.

The process is suitable for depositing water-soluble materials, water-insoluble materials, and combinations thereof. In the case of water-insoluble materials, it is acceptable to keep the aqueous solution agitated in the solution pan to avoid the settling of the material(s). It is also acceptable the use of structurants or thickening agents to promote the suspension of the insoluble materials in water. The coating can comprise a plurality of functional materials by using an aqueous solution comprising more than one functional material or by using aqueous solutions comprising different materials in different printing steps.

The fountain roller does not contact the anilox roller when transferring the aqueous solution to reduce wear. In one embodiment, the fountain roller is made of soft durometer rubber which is silicone coated. The softness permits the fountain roller to pick up the most aqueous solution possible. Fountain rollers are commercially available from Mid American Rubber.

In one embodiment, a doctor blade is used to meter the aqueous solution to a consistent thickness on the surface of the anilox roller. In another embodiment, the doctor blade is a ceramic coated metal blade like the one supplied by BTG, Norcross GA.

The anilox roller includes a multiplicity of microscopic cells that are arranged in a pattern next to each other and cover the entire surface of the roller. These cells hold the aqueous solution. The cells typically have either a honeycomb shape or a "tri-helical" pattern. The cells can be oriented in rows that run at an angle to the longitudinal axis of the roller (so that the rows of larger sized cells appear to form screw threads around the roller). Typical angles are 30, 45 and 60 degrees. As is typical in traditional printing, different colors of inks are typically printed with cells that are oriented at different angles.

The coarseness of the anilox roller determines how much of the solution is transferred to the film. As the volume of the anilox cells increases (e.g., from 60 to 100 bcm (billion parts of cube micron)), at comparable cell emptying on the plate (transfer), the volume of aqueous solution transferred on the plate and then on the substrate increases.

Anilox rollers are often made of stainless steel. However, for some applications such as the printing of acidic and corrosive materials, (for example, organic perodixes and in particular dibenzoyl peroxide), the rollers should have a ceramic coating to prevent corrosion of the stainless steel roller. Anilox rollers are commercially available from Harper Corporation of America and Interflex.

Flexographic roller is a flexible patterned roll. The flexible plate material can be a 50 durometer, 0.067 inch thick material. Other plates that can be used for flexographic printing include those identified at column 4, lines 30 to 45 of US-A-5,458,590.

The water-soluble film can be engraved or embossed such that micro (invisible to the naked eye) or macro (visible) deformations are created in a given pattern before or in conjunction with the deposition of the aqueous solution. This enables larger volumes of functional materials to be deposited, in particular when the functional materials are "sandwiched" between the two laminating films thanks to the void area created by the two engraved or embossed films coming in contact. Relatively large holes can be impressed on both films and the aqueous solution can be applied on both films surface before laminating them together. The level of functional material(s) presents between the two films is much more thanks to the voids created by joining two holes together. Embossing plates that can be used in a flexographic equipment are supplied by Trinity Graphic USA, FL. Another method of holding more functional material on the film is to pre-apply a primer that forms a micro-cellular morphology (small cells) on the film. These primers are micro-cellular coatings based on polyurethane systems that can be applied via coating and printing methods and are supplied by Crompton Corporation, CT. The macro deformations can be achieved by subjecting the film to series of intermeshing ring rolls or engraving flexographic plates. Micro deformations can be either formed by engraving rolls with micro patterns or by using a hydro formed film that has protruding shapes (e.g. hollow tubes). Protruding hollow shapes can hold additional functional material(s) when in liquid or slurry form through capillary force.

Another embodiment of printing is jet printing or ink-jet type printing. Such industrial printing techniques are known. See e.g., WO 03/091028 A1; WO 00/20157; US 5,463,416. In one embodiment, the printing does not contact the film or substrate. This technique may prove useful when wishing not to disturb the film based on manufacturing efficiencies or simply because of the presence of embossings or other ornamental features on the film or substrate. Messages, ornamental designs, pictures, and the like may also be printed using methods such as those described in WO 2005/002360. In another embodiment, graphics may be printed in 2D or 3D with UV curable dye that is water-soluble or at least partially water-soluble.

In yet another embodiment, the functional composition is a powder or substantially a solid. In this embodiment, the powder or solid composition is printed onto the film or substrate.

One aspect provides a number of ways of making water-soluble films. Casting, thermoforming, melt processes are some examples. See e.g., US 6,946,501, esp. col. 7, line 55 et seq.; US 5,272,191; and US 5,646,206. In one embodiment, the substrate comprises wafers, foam shapes, sponges, films, other three dimensional forms, or combinations thereof.

### Structurant

It is acceptable to add a structurant to the aqueous solution, especially if the functional material is insoluble in the aqueous solution because the presence of the structurant helps the suspension of the functional material. Acceptable for use herein are polymeric structurants selected from the group consisting of polyacrylates and derivatives thereof; polysaccharides and derivatives thereof; polymer gums and combinations thereof. Polyacrylate-type structurants comprise in particular polyacrylate polymers and copolymers of acrylate and methacrylate. An example of a suitable polyacrylate type structurant is Carbopol Aqua 30 available from B.F. Goodridge Company. Examples of polymeric gums which may be used as structurant herein can be characterized as marine plant, terrestrial plant, microbial polysaccharides and polysaccharide derivatives. Examples of marine plant gums include agar, alginates, carrageenan and furcellaran. Examples of terrestrial plant gums include guar gum, gum arable, gum tragacenth, karaya gum, locust bean gum and pectin. Examples of microbial polysaccharides include dextrin, gellan gum, rhamsan gum, welan gum and xanthan gum. Examples of polysaccharide derivatives include carboxymethyl cellulose, methyl hydroxypropyl cellulose, hydroxy propyl cellulose, hydroxyethyl cellulose, propylene glycol alginate and hydroxypropyl guar. The second structurant is selected from the above list or a combination thereof. Acceptable polymeric gums include pectin, alginate, arabinogalactan (gum Arabic), carrageenan, gellan gum, xanthan gum and guar gum. If polymeric gum structurant is employed herein, an acceptable material of this type is gellan gum. Gellan gum is a tetrasaccharide repeat unit, containing glucose, glucurronic acid, glucose and rhamrose residues and is prepared by fermentation of Pseudomonaselodea ATCC 31461. Gellan gum is commercially marketed by CP Kelco U.S., Inc. under the KELCOGEL tradename. The aqueous solution may comprise from about 0.1 to about 20%, alternatively from about 1 to about 10% by weight of the aqueous solution of structurant. One acceptable structurant for use herein is polyvinyl alcohol (PVA). PVA not only gives the aqueous solution the right viscosity to achieve high loadings but also acts as a binder to layer-up successive layers of the coating making a very strong, flake-free coating. In one embodiment, the level of PVA in the aqueous solution is from about 0.5 to about 20%, alternatively from about 1 to about 10%, alternatively from about 2 to about 5% by weight of the aqueous solution. Water-Soluble Films

One aspect of the invention provides for a water-soluble film. The water-soluble film is a film comprised of a polymeric material and has a water-solubility of at least 50%, alternatively at least 75%, or even at least 95%, as measured by the Water-Solubility Method as provided herein.

Water-Solubility Method: 50 grams ± 0.1 gram of film material is added in a preweighed 400 ml beaker and 245ml ± 1ml of distilled water is added. This is stirred vigorously on a magnetic stirrer set at 600 rpm, for 30 minutes. Then, the mixture is filtered through a folded qualitative sintered-glass filter with a pore size as defined above (max. 20 micron). The water is dried off from the collected filtrate by any conventional method, and the weight of the remaining material is determined (which is the dissolved or dispersed fraction). The % water-solubility is then calculated.

Acceptable polymeric materials are those which are formed into a film or sheet or laminate. The film can, for example, be obtained by casting, blow-moulding, extrusion or blown extrusion of the polymeric material, as known in the art. Examples of polymers, copolymers or derivatives thereof suitable for use as film material are chosen from polyvinyl alcohols, modified polyvinyl alcohols, polyvinyl pyrrolidone, polyvinyl alcohol copolymers such as polyvinyl alcohol/polyvinyl pyrrolidone, partially hydrolyzed polyvinyl acetate, polyalkylene oxides such as polyethylene oxide, acrylamide, acrylic acid, cellulose, alkyl cellulosics such as methyl cellulose, ethyl cellulose and propyl cellulose, cellulose ethers, cellulose esters, cellulose amides, polyvinyl acetates, polycarboxylic acids and salts, polyaminoacids or peptides, polyamides, polyacrylamide, copolymers of maleic/acrylic acids, polysaccharides including starch, modified starch, gelatin, and natural gums such as pectin, xanthan, and carrageenan, or combinations thereof. Acceptable polymers are selected from polyacrylates, especially sulfonated polyacrylates, and water-soluble acrylate copolymers, alkylhydroxy cellulosics such as methylcellulose, carboxymethylcellulose sodium, modified carboxy-methylcellulose, dextrin, ethylcellulose, propylcellulose, hydroxyethyl cellulose, hydroxypropyl methylcellulose, maltodextrin, polymethacrylates. In yet another embodiment the polyers are selected from polyvinyl alcohols, polyvinyl alcohol copolymers and hydroxypropyl methyl cellulose (HPMC), and combinations thereof. In another embodiment, the level of polymer in the film, for example a PVA polymer, is at least 60%.

Acceptable water-soluble materials which may be considered for forming the film include low molecular weight and/or chemically modified polylactides; such polymers have been produced by Chronopol, Inc. and sold under the Heplon trademark. Also useful are polymer blend compositions, for example comprising hydrolytically degradable and water-soluble polymer blend such as polylactide and polyvinyl alcohol, achieved by the mixing of polylactide and polyvinyl alcohol, typically comprising 1-35% by weight polylactide and approximately from 65% to 99% by weight polyvinyl alcohol, if the material is to be water-dispersible, or water-soluble. In one embodiment, the PVA present in the film is from 60-98% hydrolyzed, alternatively 80% to 90%, to improve the dissolution of the material.

Also included in the water-soluble polymer family are melt processable poly(vinyl) alcohol resins (PVA); such resins are produced by Texas Polymer Services, Inc., tradenamed Vinex, and are produced under license from Air Products and Chemicals, Inc. and MonoSol film produced by MonoSol LLC. Other suitable resins include poly (ethylene oxide) and cellulose derived water-soluble carbohydrates. The former are produced by Union Carbide, Inc. and sold under the tradename Polyox; the latter are produced by Dow Chemical, Inc. and sold under the Methocel trademark. Typically, the cellulose derived water-soluble polymers are not readily melt processable. One acceptable water-soluble thermoplastic resin for this application is PVA produced by MonoSol LLC. Any number or combination of PVA resins can be used. One optional grade, considering resin processability, film durability, water solubility characteristics, and commercial viability is MonoSol film having a weight average molecular weight range of about 55,000 to 65,000 and a number average molecular weight range of about 27,000 to 33,000.

Mixtures or combinations of polymers can also be used. This may in particular be beneficial to control the mechanical and/or dissolution properties of the film, depending on the application thereof and the required needs. In one embodiment, a mixture of polymers is used, having different weight average molecular weights, for example a mixture of PVA or a copolymer thereof of a weight average molecular weight of about 10,000 to about 40,000, alternatively about 20,000, and of PVA or copolymer thereof, with a weight average molecular weight of about 100,000 to about 300,000, alternatively about 150,000.

Polymer films comprising polyvinyl alcohol can be prepared that are particularly rapidly dissolvable at colder temperatures, i.e., less than about 10°C (50°F) or less than about 4,4°C (40°F). Further, polyvinyl alcohols having varying average molecular weights (i.e. mean weights of the molar masses) such as from about 6,000 to about 78,000 or higher may be used. Likewise, polyvinyl alcohol having varying degrees of hydrolysis may also be used to advantage. In one embodiment, such polymers are less than about 90%, alternatively less than about 85%, and alternatively less than about 80% hydrolyzed, but will be more than about 60% and alternatively at least about 70% hydrolyzed. Blends of water-soluble polymers having different degrees of hydrolysis may also be used to advantage. Other acceptable film-forming polymers include polyethylene oxide, polyvinyl pyrrolidone, hydroxypropyl methylcellulose and hydroxyethylcellulose.

Blends of water-soluble film-forming polymers may also be used to advantage. Blends offer additional advantages in that rapidly dissolving films can be produced with good mechanical properties for subsequent handling and converting into manufactured articles. For instance, a blend containing at least two types of water-soluble polymers that have disparate molecular weights, can be used to prepare film that is rapidly dissolving under cold water conditions. In one embodiment, such blends contain at least one type of polymer that has a molecular weight greater than about 50,000, alternatively greater than about 60,000, and alternatively greater than about 70,000, and a second polymer or mixture of polymers having an average molecular weight of less than about 30,000, alternatively less than about 15,000, and alternatively less than about 10,000.

More specifically, a blend of at least one polyvinyl alcohol having a molecular weight of about 78,000 and higher and a second polyvinyl alcohol about 6,000 or lower has been found to produce a rapidly dissolving film under cold water conditions. A low percentage of the higher molecular weight polyvinyl alcohol, namely, less than about 50% alternatively less than about 40%, and alternatively less than about 30%, will produces a film with adequate strength for converting into sachets or coatings. A higher percentage of higher molecular weight polyvinyl alcohol, namely, greater than about 50%, alternatively greater than about 60% and alternatively greater than about 70%, will provide the improved strength and elasticity that is desired for vacuum forming operations, but it should be noted that such higher percentages of high MW polymers are typically accompanied by increasingly higher dissolution times. Blends of high and low molecular weight polymers at ratios of 80/20, 60/40, and 50/50 mixtures of low to high molecular weight polyvinyl alcohol can be evaluated for specific applications.

By way of example, a rapidly dissolving film can be prepared from a blend of polyvinyl alcohol that comprises from about 60% to about 95% of polyvinyl alcohol of an average molecular weight from about 3,000 to about 30,000 and from about 5% to about 40% of polyvinyl alcohol of an average molecular weight from about 30,000 to about 200,000. The degree of hydrolysis in the polyvinyl alcohol blend can be less than about 90 mol%, alternatively less than about 85 mol%, and alternatively less than about 80 mol%. The film formed from this composition can dissolve in a beaker of water at a temperature below about 68° F in less than about 5 minutes with agitation.

In addition, blends of different types of polymer materials can also be formulated and prepared to produce the films of the present invention. For instance, ratios of 80/20, 60/40 and 50/50 with mixes of polyvinyl alcohol and polyvinyl pyrrolidone, polyvinyl alcohol and polyethylene oxide, polyvinyl alcohol and hydroxyethyl cellulose, polyvinyl pyrrolidone and hydroxyethyl cellulose, polyvinyl pyrrolidone and polyethylene oxide, and polyethylene oxide and hydroxyethyl cellulose, hydroxypropyl methylcellulose and polyvinyl alcohol, can be used to advantage.

The film comprises water-soluble materials.

Different thicknesses of film may also be used. Thicker films generally take more time to dissolve than a thinner film. In one embodiment, the overall average thickness of the substrate is less than about 1 mm, alternatively less than about 0.5 mm, alternatively less than about 0.15 mm, alternatively less than about 0.1 mm, alternatively less than about 0.05 mm, alternatively less than about 0.04 mm, alternatively greater than about 0.01 mm. In one embodiment, the average thickness of the film of the substrate comprises from about 0.025 mm to about 0.160 mm, alternatively from about 0.060 mm to about 0.130 mm. Although delayed release of perfumes is generally desired, the substrate cannot take so long dissolve as to stain laundry. The substrate should, in one embodiment, completely dissolve within the time frame of the laundry wash cycle (irrespective of water temperature). In another embodiment, if the substrate comprises wafers, foam shapes, sponges, or other three dimensional forms, then the thickness of the substrate can be greater, even up to about several centimeters in thickness; alternatively less than about 1 cm in thickness.

Typically the water-soluble film has a basis weight from about 25 g/m² to about 150 g/m², alternatively from about 50 g/m² to about 100 g/m².

In one embodiment, the water-soluble films comprises a PVA film. Suitable PVA films are known under the trade reference MonoSol M8630, as sold by MonoSol LLC of Gary, Indiana, US, and PVA films of corresponding solubility and deformability characteristics. Other films suitable for use herein include films known under the trade reference PT film or the K-series of films supplied by Aicello, or VF-HP film supplied by Kuraray.

The water-soluble film herein may comprise other additive ingredients than the polymer or polymer material and water. For example, it may be beneficial to add plasticisers, for example glycerol, ethylene glycol, diethyleneglycol, propylene glycol, sorbitol and mixtures thereof. Glycerol is one preferred plasticisers. Other useful additives include disintegrating aids.

### Insolubilizer Agents

One aspect of the invention provides for the use of film insolubilizer agents. Acceptable insolubilizer agents for use herein are salts. Salts may include organic or inorganic electrolytes. Suitable salts may include a cation or mixtures of cations

In one embodiment, the film insolubilizer agent is used in a level of from about 0.5 to about 10%, alternatively from about 1 to about 5% by weight of the aqueous solution. In another embodiment, the film insolubilising agent is a salt chosen at least one of the following from the group consisting of: sodium sulfate, sodium citrate, sodium tripolyphosphate, potassium citrate, and combinations thereof. Fabric Softening Actives

In one embodiment of the invention, the functional material may comprise a fabric softening active. Such fabric softening actives are preferably those effective in a "wash-added" (verses a rinse-added) context, although the use of quaternary ammonium compounds are not excluded as functional materials from this invention. Non-limiting examples include silicone, fatty acid, polyethylene waxes, sucrose esters, clays, and triglycerides. Coacervates with silicone and other softening actives in these co-pending patent applications can be found in: US20050020476A1 and US20060217288A1. In one embodiment, the aqueous solution is free or substantially free of a fabric softening active. Additional suitable fabric softening actives are disclosed in US 2006/0058214A1.

### Deposition Agents

In one embodiment of the invention, the functional material may also comprise deposition agents including, but not limited to I) non-quaternary materials that are (a) acyclic polymers or copolymers having nitrogen moieties in the backbone or in the pendant groups, or (b) vinyl polymers or copolymers having nitrogen heterocyclics in the pendant groups; II) non-polysaccharide polyquaterniums and other polymeric cationic quaternary materials; and mixtures thereof.

The deposition agents suitable for use herein are polymeric materials having a weight average molecular weight generally in the range from about 1000 to about 1,000,000, or from about 1000 to about 200,000, or from about 2500 to about 1,000,000, or from about 5000 to about 500,000. In some embodiments, the deposition aid is polyacrylamide or derivatives thereof, the weight average molecular weight of the deposition aid is from about 1,000,000 to about 15,000,000.

When present, each deposition agent comprises, based on total composition weight, at one of the following levels, from about 0.1% to about 20%, alternatively from about 0.2% to about 15%, alternatively from about 0.2% to about 10 wt %, and alternatively from about 0.2% to about 5%.

In some embodiments of the present invention, suitable deposition agents are acyclic polymers or copolymers derived from monomers having nitrogen moieties, including but not limited to, amine, imine, amide, imide, acrylamide, methacrylamide, amino acid, and mixtures thereof. Additional suitable deposition agents are disclosed in U.S. Patent Publication No. 2006/0058214 A1.

### Form of the fabric care product

In one embodiment, the fabric care product can be in the form of powder, liquid or gel or in unit dose form including tablets and in particular pouches, capsules and sachets. In one embodiment, the product is in a laminar or substantially planar form.

In the laundry products embodiments, the functionalized substrates can separate functional materials from one another as well as control the release of the functional materials. The functionalized substrates can comprise more than one functional material in a layer or a plurality of layers comprising a plurality of functional materials or discrete regions comprising different functional materials.

In one embodiment, the functionalized substrate comprises a first layer of water-soluble film and a second layer of water-soluble film, wherein the first layer is coated with a first functional material and the second layer is coated with a second functional material. In one embodiment, the composition of the first layer of water-soluble film is the same as the composition of the second layer of water-soluble film. In another embodiment, the composition of the first layer of water-soluble film is different than the composition of the second layer of water-soluble film. In one embodiment, the first functional material is the same as the second functional material. In another embodiment, the first functional material is different than the second functional material. Examples of different functional materials can include embodiments where the first functional material comprises a PMC and the second functional material can be a functional material other than PMC; or where the first functional material comprises a first PMC and the second functional material comprises a second PMC, wherein the encapsulated perfume components are different. These different encapsulated perfumes components can have different chemical formulas or create different scent effects. In another embodiment, the present invention comprises more than two layers, wherein each layer comprises a functional material, and wherein at least two of the layers have different functional materials.

One advantage to having more than one layer is that a relatively less expensive film can be placed around a more expensive film, thereby lowering overall cost. Additionally, certain substrates may be capable of accommodating higher amounts of certain functional materials. By providing a multi-layers system, the combination of substrate layer to functional material can be optimized so that the layer is more accommodating to a specific functional material. Benefits for multi-layered systems include, but are not limited to, providing higher loading capacity, promoting substrate stability, isolating interaction between certain functional materials, etc. Further, by placing specific functional materials in specific layers the release of the functional materials into the wash and/or rinse can be controlled. For example, functional materials which are desirably released earlier in the wash and/or rinse can be placed on the outer layer(s), whereas functional materials which are desirably released later in the wash and/or rinse can be placed in the inner layer(s).

In another embodiment, the article comprises a first functionalized substrate and a second functionalized substrate. In one embodiment, the water-soluble film of the first functionalized substrate is different than the water-soluble film of the second functionalized substrate. In another embodiment, the functional material of the first functionalized substrate is different than the functional material of the second functionalized substrate.

In an alternative embodiment, the coating of the film with two or more materials can act at the same time. In one embodiment, the two materials are placed in separate discrete regions of the film in order to avoid interaction during storage. Each material can be coloured with a dye or pigment to indicate to the user the presence of different materials. In another embodiment, the functionalized substrates of the invention are particularly suitable to separate incompatible materials thereby avoiding the interaction between the incompatible materials.

In one embodiment of the present invention the composition is in the form of a unit dose cleaning product. It could be single or multi-compartment unit dose product, optionally a vacuum- or thermoformed multi-compartment water-soluble pouch, wherein one of the compartments, optionally containing a solid powder composition. Acceptable manufacturing methods for unit dose executions are described in US 2005/0065051 A1; US 2005/ 0061703 A1. Single compartment pouches can be made by placing a first piece of film in a mould, drawing the film by vacuum means to form a pocket, filling the formed pocket with a fabric care active including the guest-host complex, and placing and sealing the formed pocket with another piece of film.

Multi-compartment pouches comprising a powder and a liquid composition can be made by placing a first piece of film in a mould, drawing the film by vacuum means to form a pocket, pinpricking the film, dosing and tamping the powder composition, placing a second piece of film over the first pocket to form a new pocket, filling the new pocket with the liquid composition, placing a piece of film over this liquid filled pocket and sealing the three films together to form the dual compartment pouch.

In one embodiment of the present invention, the article further comprises a surfactant suitable for cosmetic use on skin. Suitable surfactants include soap, anionic surfactant, nonionic surfactant, amphoteric surfactant, cationic surfactant and mixtures thereof. Further, in one embodiment, the functionalized substrate further comprises a carrier material. Suitable carrier materials include soluble or partially soluble starches, water soluble amorphous solids or semicrystalline water soluble solids, and mixtures thereof. In yet another embodiment, the article comprises a surfactant suitable for cosmetic use on skin and a Functionalized substrate comprising a carrier material and a functional material comprising a microcapsule encapsulating a perfume. In one embodiment article further comprises: propylene glycol, sorbitol, glycerin, sodium laureth sulfate, sodium stearate, sodium myristate, sodium cocoyl isethionate, triethanolamine, water, and a perfume microcapsule.

### Examples (Examples 1,3,4,6-9 are comparative examples)

Example 1: An aqueous perfume microcapsule (PMC) solution comprising:

| | |
|---|---|
| Perfume accord in capsule | 16.57% |
| Isopropyl Myristate in capsule | 16.57% |
| Urea/Formaldehyde capsule wall | 5.84% |
| Water (outer slurry) | 53.86% |
| Ethylacetoacetae (outer slurry) | 6.8% |
| Xanthan gum (outer slurry) | 0.29% |
| Direct 086 Blue dye | 0.07% |

was printed on a M8630™ 3.0 mil (100 grams per square mater basis weight) water-soluble PVA film supplied by MonoSol LLC of Gary, IN. The solution was printed on the film via a narrow web Comco flexographic printing press (commercially available from Mark Andy of Milford, Ohio), measuring 28 cm in width, having 6 stations and capable of hot air drying.

Ceramic coated anilox rolls were used (supplied by Harper Corp). Fountain rolls (that pick up the aqueous solution from the pan and transfer it to the anilox roll) are supplied by Mid American Rubber, Three Rivers, MI. Photopolymer printing plates are supplied by Du Pont (Cyrel brand). The printing took place on three of the six stations. The three sequential stations used respectively a 60 lpi (lines per inch)/40 bcm (billion cubic micron), 30 lpi/100 bcm and 30 lpi /100 bcm anilox rolls and it was allowed to dry in between the stations via convected hot air blown over the printed film surface to remove the water. The coating is in a level of 85 g/m² and the loading is 85% by weight of the uncoated film. The perfume loading is 36% of the total dried coating weight.
The final dry petal shape (cut from the film with a die cutter) was of about 2.5" x 4" rectangle, total weight of 1.6g.
Composition in weight percent:

| M8630 PVOH 3 mil film | Water-soluble film substrate | 81.25% |
|---|---|---|
| Appleton PMC | PMC mixture composition as follows: | 18.75% |
| *Perfume oil* | *Perfume* | *[6.75%]* |
| *Isopropyl Myristate* | *Perfume solvent* | *[6.75%]* |
| *Urea*/*formaldehyde* | *Capsule wall* | *[2.37%]* |
| *Ethyl Acetoacetate* | *Formaldehyde scavenger* | *[2.75%]* |
| *Xanthan gum* | *Thickening*/*suspension* | *[0.12%]* |
| *Direct Blue 086 dye* | *Product color* | *[0.01%]* |
| TOTAL | | 100.00 |

Example 2: A printing process as that described in example 1 is used but after the third printing station the drying step is eliminated and a second M8630™ film is placed over the wet printed film to create a laminate.

Example 3: Graphics are printed in a laminate obtained according to Example 2 using a white ink (Aqua HSX05700 manufactured by Environmental Inks and Coatings, Morgaton, NC).

Example 4: Graphics which change colour with temperature are printed in a laminate obtained according to Example 2 using a thermo chromic ink: Dynacolor commercially available from CTI, Colorado Springs, Colorado.

Example 5: The laminate resulting from example 2 is cut in 2.6 x 2.6 mm and 2 x 10 mm pieces and added to a liquid fabric softener composition, wherein the liquid fabric softener composition comprises a known quaternary ammonium compound.

Example 6: A printing method as described in example 1 is used but a 1.5 mil (50 grams per square meter basis weight) water-soluble film supplied by MonoSol LLC of Gary, IN is used instead and the aqueous solution contains 2.5% of PVA by weight of the solution. Two printing stations are used, employing respectively a 60 lpi/40 bcm and 30 lpi/100 bcm anilox rolls. The coating is in a level of 94 g/m² and the loading is 94% by weight of the uncoated film. The perfume loading is 66.6% of the total dried coating weight.
The final dry petal shape (cut from the film with a die cutter) was of about 2.5" x 4" rectangle, total weight of 1.4g.
Composition in weight percent:

| M8630 PVOH 3 mil film | Water-soluble film substrate | 71.30 |
|---|---|---|
| Appleton PMCs | PMC mixture composition as follows: | 28.70 |
| *Perfume oil* | *Perfume* | *[18.56]* |
| *Urea*/*formaldehyde* | *Capsule wall* | *[3.27]* |
| *Ethyl Acetoacetate* | *Formaldehyde scavenger* | *[3.48]* |
| *MgCl2* | *Electrolyte* | *[2.41]* |
| *Sanolin Violet E2R* | *Product color* | *[0.83]* |
| *Formic Acid* | *Preservative* | *[0.10]* |
| *Xanthan gum* | *Thickening*/*suspension* | *[0.05]* |
| TOTAL | | 100.00 |

Example 7: A printing method as described in example 1 is used but a 2.0 mil (67 grams per square meter basis weight) water-soluble film supplied by MonoSol LLC of Gary, IN is used instead. Four printing stations are used, employing respectively a 60 lpi/40 bcm, 30 lpi/100 bcm, 30 lpi/100 bcm and 30 lpi/100 bcm anilox rolls.

Example 8: A printing method as described in example 7 is used but a flexo OPV (Over Print Varnish) water-soluble material sold as TV96-6963 supplied by Sun Chemical Corporation of Charlotte, NC is printed in the fourth printing station instead to protect the perfume microcapsule from external moisture.

Example 9: The method described in example 6 is repeated but in this occasion the aqueous solution comprises 4 % by weight of the solution of sodium sulphate. Only two printing stations are used, employing respectively a 60 lpi/40 bcm and a 30 lpi/100 bcm anilox rolls.

It should be understood that every maximum numerical limitation given throughout this specification includes every lower numerical limitation, as if such lower numerical limitations were expressly written herein. Every minimum numerical limitation given throughout this specification includes every higher numerical limitation, as if such higher numerical limitations were expressly written herein. Every numerical range given throughout this specification includes every narrower numerical range that falls within such broader numerical range, as if such narrower numerical ranges were all expressly written herein.

All parts, ratios, and percentages herein, in the Specification, Examples, and Claims, are by weight and all numerical limits are used with the normal degree of accuracy afforded by the art, unless otherwise specified.

The dimensions and values disclosed herein are not to be understood as being strictly limited to the exact numerical values recited. Instead, unless otherwise specified, each such dimension is intended to mean both, the recited value and a functionally equivalent range surrounding that value. For example, a dimension disclosed as "40 mm" is intended to mean "about 40 mm".

The citation of any document is not to be construed as an admission that it is prior art with respect to the present invention. To the extent that any meaning or definition of a term in this written document conflicts with any meaning or definition of the term in a document, the meaning or definition assigned to the term in this written document shall govern.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. An article comprising a functionalized substrate comprising at least two substantially planar water-soluble films carrying a coating of a functional composition, wherein the coating is between the two films, and wherein the functional composition comprises a friable microcapsule, wherein the microcapsule comprises a perfume microcapsule; wherein the coating is in a level of at least 5 g/m², alternatively at least 10 g/m², alternatively at least 50g/m², and alternatively at least 100 g/m².

2. The article according to any of the preceding claims wherein the functionalized substrate comprises a coating loading of at least 10%, alternatively at least 20%, alternatively at least 30%, alternatively at least 40%, alternatively at least 50%, alternatively at least 100%.

3. The article according to any of the preceding claims, wherein the functionalized substrate has a maximum linear dimension of from 0.2 mm to 100 mm, alternatively from 0.5 mm to 50 mm, and alternatively from 1 mm to 20 mm.

4. The article according to any of the preceding claims, wherein the functionalized substrate has an average thickness of less than 1 mm, alternatively less than 0.5 mm, alternatively less than 0.15 mm, alternatively less than 0.1 mm, alternatively less than 0.05 mem, alternatively less than 0.04 mm, alternatively greater than 0.01 mm.

5. The article according to any of the preceding claims, wherein the coating is applied on the film by means of any coating process, including spray, knife, rod, kiss, slot, painting, printing and mixtures thereof.

6. The article according to any of the preceding claims, wherein the functionalized substrate is cut or formed into a shape wherein the shape and the perfume are coordinated.

7. A process of making a functionalized substrate according to any of the preceding claims comprising the steps of printing to at least one side of the film to form the coating.

## Patentansprüche

1. Artikel, der ein funktionalisiertes Substrat umfasst, das mindestens zwei im Wesentlichen ebene wasserlösliche Folien umfasst, die eine Beschichtung einer funktionellen Zusammensetzung tragen, wobei die Beschichtung zwischen den zwei Folien ist und wobei die funktionelle Zusammensetzung eine zerreibbare Mikrokapsel umfasst, wobei die Mikrokapsel eine Duftstoff-Mikrokapsel umfasst, wobei die Beschichtung in einem Anteil von mindestens 5 g/m², als Alternative mindestens 10 g/m², als Alternative mindestens 50 g/m² und als Alternative mindestens 100 g/m² vorliegt.

2. Artikel nach einem der vorstehenden Ansprüche, wobei das funktionalisierte Substrat eine Beschichtungsbeladung von mindestens 10 %, als Alternative mindestens 20 %, als Alternative mindestens 30 %, als Alternative mindestens 40 %, als Alternative mindestens 50 %, als Alternative mindestens 100 % umfasst.

3. Artikel nach einem der vorstehenden Ansprüche, wobei das funktionalisierte Substrat eine maximale lineare Abmessung von 0,2 mm bis 100 mm, als Alternative von 0,5 mm bis 50 mm und als Alternative von 1 mm to 20 mm aufweist.

4. Artikel nach einem der vorstehenden Ansprüche, wobei das funktionalisierte Substrat eine durchschnittliche Dicke von weniger als 1 mm, als Alternative weniger als 0,5 mm, als Alternative weniger als 0,15 mm, als Alternative weniger als 0,1 mm, als Alternative weniger als 0,05 mm, als Alternative weniger als 0,04 mm, als Alternative mehr als 0,01 mm aufweist.

5. Artikel nach einem der vorstehenden Ansprüche, wobei die Beschichtung mittels eines beliebigen Beschichtungsverfahrens, einschließlich Sprüh-, Messer-, Stab-, Übertragungswalzen-, Schlitzdüsen-, Streich-, Druckverfahren und Mischungen davon, auf die Folie aufgetragen wird.

6. Artikel nach einem der vorstehenden Ansprüche, wobei das funktionalisierte Substrat in einer Form geschnitten oder ausgebildet wird, wobei die Form und der Duftstoff koordiniert sind.

7. Herstellungsverfahren für ein funktionalisiertes Substrat nach einem der vorstehenden Ansprüche, das die Schritte des Druckens auf mindestens eine Seite der Folie umfasst, um die Beschichtung zu bilden.

## Revendications

1. Article comprenant un substrat fonctionnalisé comprenant au moins deux films hydrosolubles essentiellement plans portant un revêtement d'une composition fonctionnelle, dans lequel le revêtement est entre les deux films, et dans lequel la composition fonctionnelle comprend une microgélule friable, dans lequel la microgélule comprend une microgélule de parfum ; dans lequel le revêtement est à un taux d'au moins 5 g/m², en variante au moins 10 g/m², en variante au moins 50 g/m², et en variante au moins 100 g/m².

2. Article selon l'une quelconque des revendications précédentes, dans lequel le substrat fonctionnalisé comprend un chargement de revêtement d'au moins 10 %, en variante au moins 20 %, en variante au moins 30 %, en variante au moins 40 %, en variante au moins 50 %, en variante au moins 100 %.

3. Article selon l'une quelconque des revendications précédentes, dans lequel le substrat fonctionnalisé a une dimension linéaire maximale allant de 0,2 mm à 100 mm, en variante de 0,5 mm à 50 mm, et en variante de 1 mm à 20 mm.

4. Article selon l'une quelconque des revendications précédentes, dans lequel le substrat fonctionnalisé a une épaisseur moyenne de moins de 1 mm, en variante moins de 0,5 mm, en variante moins de 0,15 mm, en variante moins de 0,1 mm, en variante moins de 0,05 mm, en variante moins de 0,04 mm, en variante plus de 0,01 mm.

5. Article selon l'une quelconque des revendications précédentes, dans lequel le revêtement est appliqué sur le film au moyen de n'importe quel procédé de revêtement, y compris une pulvérisation, un couteau, une tige, un effleurement, une encoche, une peinture, une impression et leurs mélanges.

6. Article selon l'une quelconque des revendications précédentes, dans lequel le substrat fonctionnalisé est coupé ou formé en une forme, la forme et le parfum étant coordonnés.

7. Procédé de fabrication d'un substrat fonctionnalisé selon l'une quelconque des revendications précédentes comprenant les étapes consistant à imprimer sur au moins un côté du film pour former le revêtement.
